(19) Europäisches Patentamt
European Patent Office
Office européen des brevets

(11) Veröffentlichungsnummer: **0 213 499 A2**

(12) **EUROPÄISCHE PATENTANMELDUNG**

(21) Anmeldenummer: 86111239.9

(22) Anmeldetag: 14.08.86

(51) Int. Cl.4: C07K 5/06 , A61K 37/02

(30) Priorität: 22.08.85 DE 3529960

(43) Veröffentlichungstag der Anmeldung: 11.03.87 Patentblatt 87/11

(84) Benannte Vertragsstaaten: AT BE CH DE FR GB IT LI LU NL SE

(71) Anmelder: BOEHRINGER INGELHEIM KG
D-6507 Ingelheim am Rhein(DE)BECHDEFRITLILUNLSEAT
Anmelder: Boehringer Ingelheim International G.m.b.H
D-6507 Ingelheim am Rhein(DE)GB

(72) Erfinder: Schnorrenberg, Gerd, Dr.
Ernst-Ludwig-Strasse 66a
D-6535 Gau-Algesheim(DE)
Erfinder: Roos, Otto, Dr.
Elsheimerstrasse 36
D-6501 Schwabenheim(DE)
Erfinder: Lösel, Walter, Dr.
Im Herzenacker 26
D-6535 Gau-Algesheim(DE)
Erfinder: Wiedemann, Ingrid, Dr.
Dudenstrasse 16
D-6200 Wiesbaden(DE)
Erfinder: Gaida, Wolfram, Dr.
Selztalstrasse 77b
D-6507 Ingelheim/Rhein(DE)
Erfinder: Hoefke, Wolfgang, Dr.
Rosselstrasse 27
D-6200 Wiesbaden(DE)
Erfinder: Arndts, Dietrich, Dr.
Mühlstrasse 7
D-6531 Appenheim(DE)
Erfinder: Streller, Ilse, Dr.
Waldstrasse 16
D-6534 Stromberg(DE)

(54) **Aminosäure-Derivate, Verfahren zu ihrer Herstellung und Verwendung.**

(57) Aminosäure-Derivate der allgemeinen Formel

EP 0 213 499 A2

Xerox Copy Centre

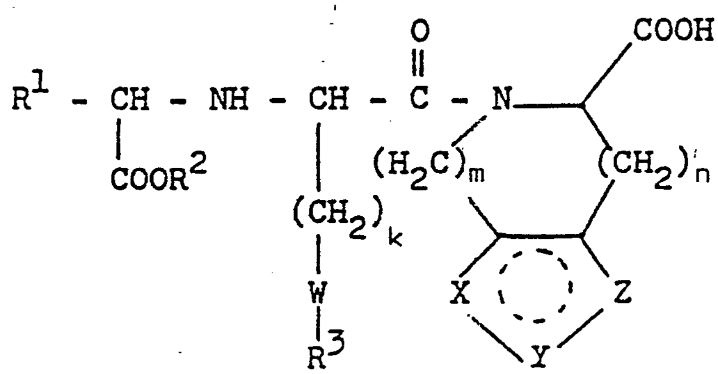

(I)

(die Bedeutung der verschiedenen Symbole ist in der Beschreibung erklärt) eignen sich zur Behandlung des Bluthochdruckes und zur Cardioprotektion. Die Herstellung und Verwendung der neuen Verbindungen erfolgt in üblicher Weise.

## Aminosäure-Derivate, Verfahren zu ihrer Herstellung und Verwendung

Gegenstand der Erfindung sind Aminosäure-Derivate der allgemeinen Formel I und ihre Salze sowie Verfahren zu ihrer Herstellung, ihre pharmazeutischen Zusammensetzungen und ihre Anwendung als Arzneimittel.

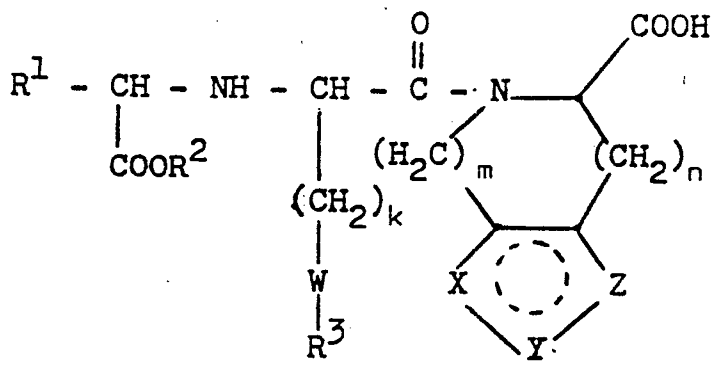

(I)

In dieser Formel bedeuten:

$R^1$ Wasserstoff, oder eine ggf. phenylsubstituierte Alkylgruppe mit 1 bis 6 Kohlenstoffatomen;

$R^2$ Wasserstoff oder eine ggf. phenylsubstituierte Alkylgruppe mit 1 bis 4 Kohlenstoffatomen;

$R^3$ Wasserstoff, eine Alkylgruppe mit 1 bis 7 Kohlenstoffatomen, die endständig eine Aminogruppe oder einen Phenylring tragen kann, oder die Gruppe $R^4$ -CO -;

$R^4$ eine Alkyl-oder Alkoxygruppe mit 1 bis 5 Kohlenstoffatomen, die, im Falle W = NH, in α-Stellung eine Aminogruppe tragen kann;

W Sauerstoff, Schwefel oder die NH-Gruppe;

n und m jeweils 0,1 oder 2, wobei die Summe n und m 1 oder 2 ist;

k 1, 2, 3 oder 4;

X, Y und Z Sauerstoff, Schwefel, $NR^5$, $CR^6$, $CHR^6$,

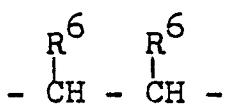 oder 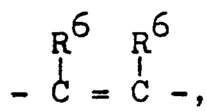,

mit der Maßgabe, daß nur einer der Reste X, Y und Z O, S,

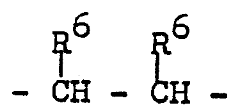 oder 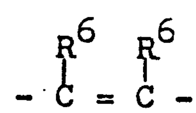

und ein oder zwei der Reste X, Y und Z $NR^5$ bedeuten können;

$R^5$ Wasserstoff oder eine Alkylgruppe mit 1 bis 4 Kohlenstoffatomen und

$R^6$ Wasserstoff oder zusammen mit einem vicinal stehenden Rest $R^6$ einen Phenylring.

Die an die Pyrrolidin-beziehungsweise Piperidincarbonsäure ankondensierten Fünf-oder Sechsringheterocyclen können gesättigt oder ungesättigt sein. Bevorzugte Heterocyclen sind: Furan, Pyrrol, Thiophen, Benzofuran, Indol, Benzothiophen, Oxazol, Imidazol, Thiazol, Isoxazol, Pyrazol, Pyrrolidin, Tetrahydrofuran, Tetrahydrothiophen, Pyridin, Pyridazin, Chinolin, Isochinolin oder Piperidin.

Die neuen Verbindungen weisen im allgemeinen mehrere Asymmetriezentren auf und liegen daher als Diastereomere oder in Form ihrer Racemate beziehungsweise ihrer racemischen Gemische vor. Die Erfindung umfaßt sowohl die racemischen Gemische als auch die einzelnen Diastereomeren. Bevorzugt sind Diastereomere, bei denen das zentrale und das zu $R_1$ benachbarte Asymmetriezentrum in S-Konfiguration vorliegt. Das an den Stickstoff-Heterocyclus gebundene Asymmetriezentrum kann dabei sowohl in der R-als auch in der S-Konfiguration vorliegen. Durch Variation der Stereochemie an diesem Asymmetriezentrum können die Wirkungsprofile in Hinblick auf die blutdrucksenkende Wirkung und/oder auf die cardioprotektive Wirkung hin beeinflußt werden.

Die Verbindungen der Formel I können als innere Salze oder, falls freie Carboxylgruppen vorhanden sind, als Alkali-oder Erdalkalisalze z.B. als Natrium-, Kalium-, Magnesium-oder Calciumsalz und als physiologisch unbedenkliche Salze mit Aminen wie Trimethylamin oder Dicyclohexylamin vorliegen. Ferner kann eine vorhandene freie Aminogruppe mit einer Mineralsäure, wie Salzsäure oder Bromwasserstoffsäure, oder einer organischen Säure, beispielsweise Essigsäure, zu einem Salz umgesetzt werden.

Bevorzugte Verbindungen der Formel I entsprechen der Formel

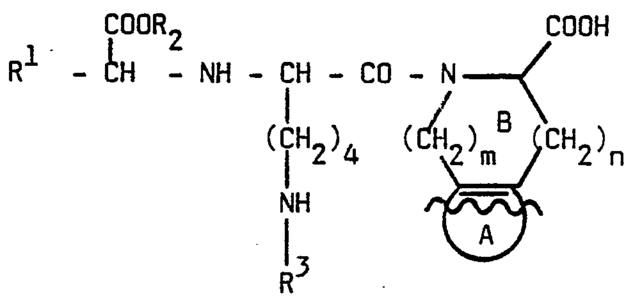

(Ia)

in der

$R^2$, $R^3$ die obige Bedeutung haben,

m 1 oder 2,

n 0 oder 1 bedeutet, wobei die Summe m + n gleich 2 ist,

A für einen der Reste

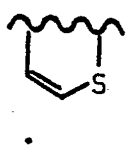

,

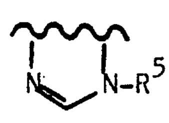

oder

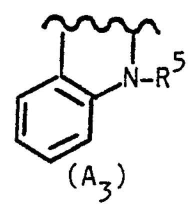

$(A_1)$ $(A_2)$ $(A_3)$

steht, wobei die Reste $A_1$, $A_2$ und $A_3$ auf beiderlei Weise mit dem Ring B verknüpft sein können, und

$R^5$ wie oben definiert ist.

Besonders hervorzuheben sind die Verbindungen der Formel

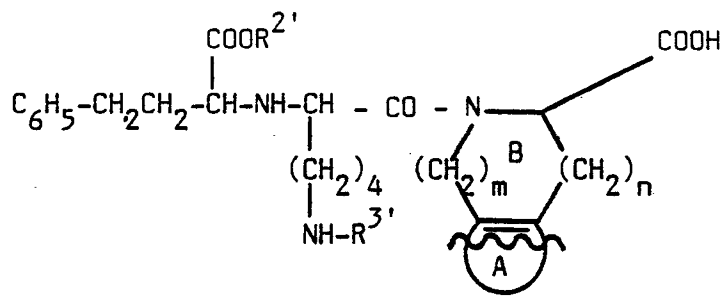

(Ib)

in der

$R^{2'}$ H oder $C_1$-$C_4$-Alkyl und

$R^{3'}$ H oder $COR^4$

bedeutet, wobei $R^4$ wie oben definiert ist,

m 1 oder 2,

n 0 oder 1 bedeutet, wobei die Summe m + n gleich 2 ist, und

A' für einen der Reste

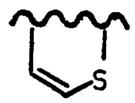

,

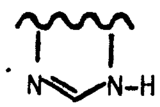

oder

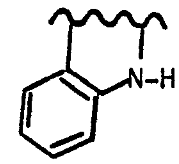

$(A_1)$ $(A_{2'})$ $(A_{3'})$

steht, wobei die Reste $A_1$, $A_{2'}$ und $A_{3'}$ auf beiderlei Weise mit dem Ring B verknüpft sein können.

Speziell seien genannt die Verbindungen

N-[N-(1(S)-Ethoxycarbonyl-3-phenylpropyl)-L-lysyl]-4,5,6,7-tetrahydro-thieno[3,2-c]pyridin-4(S)-carbonsäure

N-[N-(1(S)-Ethoxycarbonyl-3-phenylpropyl)-L-lysyl]-4,5,6,7-tetrahydro-thieno[2,3-c]pyridin-7(S)-carbonsäure

N-[N-(1(S)-Carboxy-3-phenylpropyl)-L-lysyl]-4,5,6,7-tetrahydro-thieno[2,3-c]pyridin-5(S)-carbonsäure

N-[N-(1(S)-Carboxy-3-phenylpropyl)-L-lysyl]-4,5,6,7-tetrahydro-thieno[3,2-c]pyridin-6(S)-carbonsäure

N-[N-(1(S)-Carbethoxy-3-phenylpropyl)-L-lysyl]-4,5,6,7-tetrahydro-thieno[3,2-c]pyridin-5(S)-carbonsäure

N-[N-(1(S)-Ethoxycarbonyl-3-phenylpropyl)-2-L-lysyl]-4,5,6,7-tetrahydro-thieno[2,3-c]pyridin-5(S)-carbonsäure

N-[N-(1(S)-Carbethoxy-3-phenylpropyl)-L-lysyl]-4,5,6,7-tetrahydro-thieno[3,2-c]pyridin-6(S)-carbonsäure.

Die neuen Stoffe der allgemeinen Formel I können nach verschiedenen Verfahren erhalten werden:

a) Durch Umsetzung einer Verbindung der allgemeinen Formel

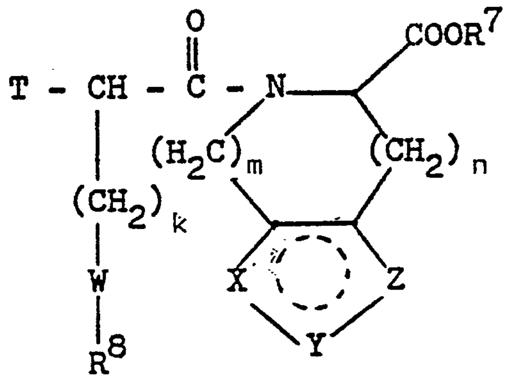

(II)

mit einer Verbindung der allgemeinen Formel III

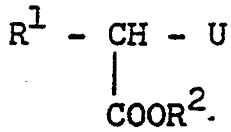

(III)

in denen

T eine nucleofuge Gruppe und

U eine Aminogruppe oder umgekehrt T eine Aminogruppe und U eine nucleofuge Gruppe,

$R^7$ Wasserstoff, eine Alkylgruppe mit 1 bis 4 Kohlenstoffatomen, die Benzylgruppe oder die Trimethylsilylgruppe,

$R^8$ die Bedeutung von $R^3$ oder, im Falle, daß W für -NH oder W für -S -und $R^3$ für -H steht, eine der Schutzgruppen tert. Butyloxycarbonyl, Benzyloxycarbonyl, Fluorenylmethyloxycarbonyl, Benzyl oder Trityl bedeutet und die Reste $R^1$, $R^2$, $R^3$, W, k, n, m, X, Y und Z die oben angeführte Bedeutung besitzen.

Als Nucleofuge können Halogenide dienen, so daß man beispielsweise jeweils von 2-Halogencarbonsäurederivaten und 2-Aminosäurederivaten ausgehen kann. Die Reaktion wird bevorzugt in polaren Lösungsmitteln wie Wasser, Alkoholen, Dimethylformamid, Acetonitril, Dimethylsulfoxid oder deren Gemischen, gegebenenfalls in Anwesenheit von Alkali-oder Erdalkalicarbonaten, tertiären Aminen, quartären Ammoniumhydroxiden oder Tetraalkylguanidinen durchgeführt. In Abhängigkeit vom verwendeten Lösungsmittel wird dabei eine Inverson der Halogencarbonsäurederivate beobachtet, die deshalb bevorzugt in der R-Konfiguration vorliegend eingesetzt werden sollten. Nach Abspaltung von $R^7$ und/oder $R^8$ nach den üblichen Verfahren, beispielsweise durch saure oder alkalische Verseifung des Esters oder katalytische Hydrogenolyse, werden die Endprodukte der allgemeinen Formel I erhalten.

b) Durch Umsetzung eines α-Oxocarbonsäurederivats der allgemeinen Formel IV,

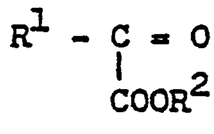

(IV)

worin $R^1$ und $R^2$ die oben erwähnte Bedeutung haben, mit einem unter a) beschriebenen Aminosäureamid der allgemeinen Formel II ($T = NH_2$) zum entsprechenden Imin, das reduziert wird. Geeignete Lösungsmittel für diese Reaktion sind Wasser oder Alkohole, aber auch unpolare Lösungsmittel wie Benzol oder Toluol. Bei Verwendung von wasserfreien Lösungsmitteln kann das Reaktionswasser durch Zugabe von Molekularsieb gebunden werden. Die Reduktion kann zum Beispiel durch Natriumborhydrid, Natriumcyanborhydrid oder katalytische Hydrierung mit Palladium auf Kohle oder Raney-Nickel als Katalysatoren erfolgen.

Bei dieser Umsetzung muß für W = NH oder für W = S und $R^3$ = H das Wasserstoffatom ebenfalls durch eine der Schutzgruppen $R^8$ ersetzt werden.

Die α-Aminocarbonsäurederivate der allgemeinen Formel II (T = $NH_2$) werden bevorzugt in der S-konfigurierten Form eingesetzt. Im Fall wird in Abhängigkeit von k, n, m, W, X, Y, Z, $R^7$ und $R^8$ eine asymmetrische Induktion am noch entstehenden Chiralitätszentrum beobachtet. Ist diese Induktion nicht vollständig, kann durch die üblichen Trennverfahren, bevorzugt durch fraktionierte Kristallisation und chromatographische Diastereomerentrennung die R-oder die S-Form rein erhalten werden.

Durch Abspaltung von $R^7$ und/oder $R^8$ nach den üblichen Verfahren, wie beispielsweise geschildert, werden die Endprodukte der allgemeinen Formel I erhalten.

c) Ausgehend von einer Verbindung der allgemeinen Formel V

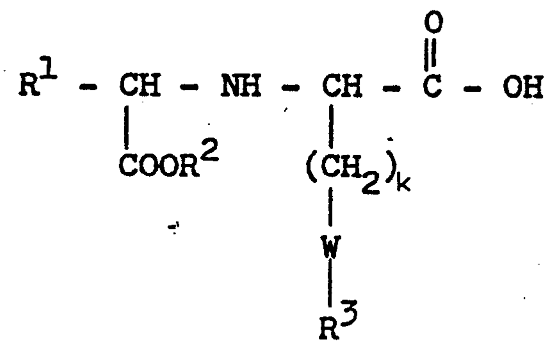

(V)

worin

$R^1$, $R^2$, $R^3$, W und k die oben angegebene Bedeutung haben, durch Umsetzung mit einer Aminosäure der Formel VI

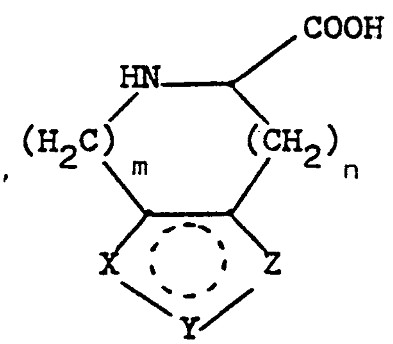

(VI)

worin

die Reste X, Y und Z sowie n und m die oben angegebene Bedeutung haben, beziehungsweise durch Umsetzung mit einem Ester einer Verbindung der allgemeinen Formel VI ($R^7$ anstelle von Wasserstoff).

Bei dieser Umsetzung muß für W = NH und W = S und $R^3$ = H das Wasserstoffatom ebenfalls durch eine der Schutzgruppen $R^8$ ersetzt werden.

Die Kondensation kann nach in Houben-Weyl, Methoden der Organischen Chemie, Bd. 15, beschriebenen Methoden erfolgen. Bevorzugtes Kondensationsmittel für die Reaktion ist N,N'-Dicyclohexylcarbodiimid, bevorzugte Carboxylschutzgruppe für die Aminosäure VI ist die tert.-Butyl-, Methyl-oder Trimethylsilylgruppe. Nach erfolgter Kondensation werden diese und $R^8$ nach den üblichen Verfahren, wie beispielsweise geschildert, abgespalten.

Die Ausgangsstoffe können nach an sich bekannten Methoden erhalten werden.

Die Ausgangsverbindungen II (T = Halogen) sind durch Kondensation von Estern von Aminosäuren der allgemeinen Formel VI mit 2-Halogencarbonsäuren über deren entsprechende

Säurechloride, gemischten Anhydride, Aktivester oder über andere in Houben-Weyl, Methoden der Organischen Chemie, Bd. 15, beschriebenen Methoden zugänglich.

Die Verbindungen II ($T = NH_2$) werden durch Reaktion von Estern der Aminosäuren mit N-geschützten Aminocarbonsäuren erhalten. Als Aminoschutzgruppen und Kondensationsmittel werden in Houben-Weyl, Methoden der Organischen Chemie, Bd. 15, beschriebene verwendet. Bevorzugt als Aminoschutzgruppen werden die -t-Butyloxycarbonyl-oder die Fluorenylmethoxycarbonylgruppe und als Kondensationsmittel N,N'-Dicyclohexylcarbodiimid verwendet.

Die Ausgangsverbindungen der allgemeinen Formel V werden durch Umsetzung eines α-Oxocarbonsäurederivats der allgemeinen Formel IV, worin $R^1$ und $R^2$ die oben erwähnte Bedeutung haben, mit einem Aminosäurederivat der allgemeinen Formel VII,

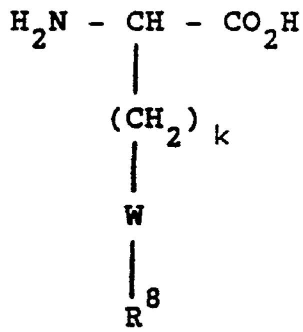

VII

worin k, W und $R^8$ die oben angegebene Bedeutung besitzen, zum entsprechenden Imin umgesetzt und dann reduziert. Geeignete Lösungsmittel für diese Reaktion sind Wasser, Alkohole, Alkohol-Wasser-Gemische, aber auch unpolare Lösungsmittel, wie z.B. Toluol. Bei Verwendung von wasserfreien Lösungsmitteln kann das Reatkionswasser durch Zugabe von Molekularsieb gebunden werden. Die Reaktion kann durch Natriumborhydrid, Natriumcyanborhydrid oder katalytische Hydrierung mit Palladium auf Kohle oder Raney-Nickel als Katalysatoren erfolgen.

Bei dieser Umsetzung muß für W = NH oder W = S und $R^3$ = H das Wasserstoffatom durch eine der Schutzgruppen $R^8$ ersetzt werden.

Die α-Aminocarbonsäurederivate der allgemeinen Formel VII werden bevorzugt in der S-konfigurierten Form eingesetzt. In diesem Fall wird in Abhängigkeit der Bedeutung von k, W und $R^8$ eine asymmetrische Induktion am neu entstehenden Chiralitätszentrum beobachtet. Ist diese Induktion nicht vollständig, kann durch die üblichen Trennverfahren, bevorzugt durch fraktionierte Kristallisation und chromatographische Diastereomerentrennung die R-oder die S-Form rein erhalten werden.

Durch Abspaltung von $R^8$ nach den üblichen Verfahren, wie beispielsweise geschildert, werden die Endprodukte der allgemeinen Formel I erhalten.

Ausgangsverbindungen der allgemeinen Formel VI können, je nach Bedeutung der Reste X, Y und Z, erhalten werden beispielsweise durch Umsetzung von

a) Tryptamin und Glyoxylsäure

(B. T. Ho et al, J. Pharm. Sci. 57, 269-274 (1968))

b) Thiophen-2-ethylamin und Glyoxylsäure

(J. P. Moffrand, Heterocycles 16, 35-37 (1981))

c) Tryptophan und Formaldehyd

(D. G. Harvey et al, J. Chem. Soc. 1941, 153-159)

d) Histidin und Formaldehyd

(M. Cain et al, Heterocycles 19, 1003-1007 (1982))

e) aus R-oder S-β-2-Thienyllalanin und Formaldehyd analog der unter c) beschriebenen Literaturvorschrift.

Bei den vorstehend beschriebenen Verfahren können die Ausgangsverbindungen in Form ihrer racemischen Gemische, ihrer Diastereomeren oder Enantiomeren vorliegen. Liegen die racemischen Gemische vor, können aus den Reaktionsprodukten nach den üblichen Verfahren wie fraktionierte Kristallisation oder chromatographische Verfahren die sterisch einheitlichen Formen angereichert oder rein erhalten werden.

Nach den vorstehend beschriebenen Verfahren können beispielsweise erhalten werden:

N-[N-(1(S)-Ethoxycarbonyl-3-phenylpropyl)-L-lysyl]-4,5,6,7-tetrahydro-thieno[3,2-c]pyridin-4(S)-carbonsäure

N-[N-(1(S)-Carboxy-3-phenylpropyl)-L-lysyl]-4,5,6,7-tetrahydro-thieno[3,2-c]pyridin-4(S)-carbonsäure

N-[N-(1(S)-Ethoxycarbonyl-3-phenylpropyl)-L-lysyl]-4,5,6,7-tetrahydro-thieno[2,3-c]pyridin-7(S)-carbonsäure

N-[N-(1(S)-Carboxy-3-phenylpropyl)-L-lysyl]-4,5,6,7-tetrahydro-thieno[2,3-c]pyridin-7(S)-carbonsäure

N-[N-(1(S)-Carboxy-3-phenylpropyl)-L-lysyl]-2,3,4,9-tetrahydro-1H-pyrido[3,4-b]indol-3(S)-carbonsäure

N-[N-(1(S)-Carboxy-3-phenylpropyl)-L-lysyl]-4,5,6,7-tetrahydro-1H-imidazo[4,5-c]pyridin-6(S)-carbonsäure

N-[N-(1(S)-Carboxy-3-phenylpropyl)-L-lysyl]-4,5,6,7-tetrahydro-thieno[2,3-c]pyridin-5(S)-carbonsäure

N-[N-(1(S)-Carboxy-3-phenylpropyl)-L-lysyl]-4,5,6,7-tetrahydro-thieno[3,2-c]pyridin-6 (S)-carbonsäure

N-[N-(1(R,S)-Carboxy-3-phenylpropyl)-L-seryl]-4,5,6,7-tetrahydro-thieno[2,3-c]pyridin-7(R,S)-carbonsäure

N-[N-(1(R,S)-Carboxy-3-phenylpropyl)-L-S-benzyl-cysteinyl]-4,5,6,7-tetrahydro-thieno[3,2-c]pyridin-4-(R,S)-carbonsäure

N-[N-(1(R,S)-Ethoxycarbonyl-3-phenylpropyl)-L-o-acetyl-seryl]-4,5,6,7-tetrahydro-thieno[3,2-c]pyridin-4(R,S)-carbonsäure

N-[N-(1(S)-Carboxy-3-phenylpropyl)-L-($N^{\epsilon}$-glycyl)-lysyl]-4,5,6,7-tetrahydro-thieno[3,2-c]pyridin-4(R,S)-carbonsäure

N-[N-(1(R,S)-Carboxy-3-phenylpropyl)-L-diamino-proprionyl]-4,5,6,7-tetrahydro-thieno[2,3-c]pyridin-5-(R,S)-carbonsäure

N[$N^{\alpha}$-(1(S)-Carboxy-3-phenylpropyl)-L-($N^{\epsilon}$-tert.-butyloxycarbonyl)-lysyl]-4,5,6,7-tetrahydro-thieno-[3,2-c] pyridin-6(S)-carbonsäure

N-[$N^{\alpha}$-(1(S)-Carboxy-3-phenylpropyl)-L-($N^{\epsilon}$-tert.-butyloxycarbonylglycyl)-lysyl]-4,5,6,7-tetrahydro-thieno-[3,2-c]pyridin-6(R,S)-carbonsäure.

N-[$N^{\alpha}$-(1(R,S)-Carboxy-3-phenylpropyl)-L-($N^{\epsilon}$-propionyl)-lysyl]-4,5,6,7-tetrahydro-thieno[3,2-c]pyridin-6(R,S)-carbonsäure

N-[N-(1(S)-Carboxy-3-phenylpropyl)-L-lysyl]-4,5,6,7-tetrahydro-thieno[3,2-c]pyridin-6(R)-carbonsäure.

Die neuen Endprodukte der allgemeinen Formel I besitzen eine starke, langanhaltende blutdrucksenkende Wirkung. Diese beruht auf einer Hemmung des Angiotensin I Converting Enzyms und damit einer Blockierung der Bildung des Vaso=konstriktors Angiotensin II aus Angiotensin I. Darüber hinaus wirken die neuen Verbindungen hemmend auf das für den Bradykininabbau verantwortliche Enzym Kininase II, das als indentisch mit dem oben genannten Converting Enzym gilt. Da Bradykinin eine gefäßerweiternde Wirkung besitzt, wird der blutdrucksenkende Effekt durch diese zusätzliche Wirkung verstärkt. Die durch Bradykinin erzeugte Blutdrucksenkung an normalen Ratten wird durch die neuen Verbindungen verstärkt.

Aufgrund ihres Wirkungsspektrums eignen sich die erfindungsgemäßen Verbindungen auch zur Infarktprophylaxe und zur Ökonomisierung des myocardialen Sauerstoffbedarfs.

Für die Hemmung des isolierten Angiotensin Converting Enzyms wurden folgende Werte erhalten:

| Verbindung nach Beispiel | $IC_{50}$ [M] |
|---|---|
| 2 | $3,5 \times 10^{-9}$ |
| 4 | $1,8 \times 10^{-8}$ |
| 7 | $1,4 \times 10^{-8}$ |
| 10 | $2,9 \times 10^{-9}$ |

Zur Blutdrucksenkung kann die Anwendung intravenös, subkutan oder peroral erfolgen. Die Dosierung bei peroraler Gabe liegt bei 2 -200 mg je Einzeldosis. Bei intravenöser Gabe oder bei gleichzeitiger Verabreichung mit Diuretika ist eine Herabsetzung der Dosis angebracht.

Daneben weisen die neuen Substanzen eine deutliche cardioprotektive Wirkung auf, die wie folgt bestimmt wurde:

Bekanntlich ist der myocardiale $Ca^{++}$-Gehalt ein Maß für die hypoxische bzw. die durch toxische Catecholamindosen hervorgerufene Herzschädigung (Higgins et al., MOL. CELL. CARDIOL. 10: 427-438, 1984; NAKANISHI et al., AM. J. PHYSIOL. 242: 437-449, 1982; FLECKENSTEIN A., Vorträge der Erlanger Physiol. Tagung 1970, Edit. KEIDEL, Springer Verl. Berlin, Heidelberg, New York, 1971). Umgekehrt ist die Inhibition der hypoxischen oder Isoprenalin-bedingten myocardialen Calciumaufnahme ein Maß für die cardioprotektive Effektivität von Calciumantagonisten - (FLECKENSTEIN s.o.), von Calmodulinhibitoren - (HIGGINS) und anderen Pharmaka, z.B. Betaadrenolytika (ARNDTS, ARZNEIMITTEL FORSCH. 25: 1279-1284, 1975).

Die cardioprotektive Wirkung wurde an wachen Ratten nach subcutaner oder peroraler Wirkstoffgabe anhand der von ARNDTS (s.o.) beschriebenen Methode festgestellt und die Wirkungsstärke der Testsubstanzen als $H_{50}$-Wert angegeben; dieser Wert entspricht der Dosis, die die durch eine Gabe von 30 mg/kg s.c. Isoprenalin bedingte myocardiale Radiocalciumaufnahme zu 50 % hemmt.

Hier erwiesen sich die untersuchten neuen Verbindungen als weit wirksamer als das bekannte Handelsprodukt Nifedipin.

Für eine cardioprotektive Wirkung kann die Anwendung intravenös, subkutan oder peroral erfolgen. Die Dosierung bei peroraler Gabe liegt bei 2 - 200 mg je Einzeldosis.

Für die Anwendung in der Therapie werden die neuen Verbindungen mit üblichen pharmazeutischen Füll-oder Trägerstoffen, Streck-, Spreng-, Binde-, Gleit-, Dickungs-oder Verdünnungsmitteln gemischt.

Als pharmazeutische Zubereitungsformen kommen zum Beispiel Tabletten, Kapseln, Zäpfchen, Lösungen, Säfte, Emulsionen oder dispersible Pulver in Frage, wobei gewünschtenfalls weitere bekannte Wirkstoffe, z.B. Saluretika, Diuretika und/oder Antihypertonika zugefügt werden können.

Entsprechende Tabletten können beispielsweise durch Mischen des oder der Wirkstoffe mit bekannten Hilfsstoffen, beispielsweise inerten Verdünnungsmitteln, wie Calciumcarbonat, Calciumphosphat oder Milchzucker, Sprengmitteln, wie Maisstärke oder Alginsäure, Bindemitteln, wie Stärke oder Gelatine, Schmiermitteln, wie Magnesiumstearat oder Talk, und/oder Mitteln zur Erzielung des Depoteffektes, wie Carboxypolymethylen, Carboxymethylcellulose, Celluloseacetatphtalat, oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Zur Erzielung eines Depoteffektes oder zur Vermeidung von Inkompatibilitäten kann der Kern auch aus mehreren Schichten bestehen. Desgleichen kann auch die Drageehülle zur Erzielung eines Depoteffektes aus mehreren Schichten bestehen, wobei die oben bei den Tabletten erwähnten Hilfsstoffe verwendet werden können.

Säfte der erfindungsgemäßen Wirkstoffe beziehungsweise Wirkstoffkombinationen können zusätzlich noch ein Süßungsmittel, wie Saccharin, Cyclamat, Glycerin oder Zucker sowie ein geschmacksverbesserndes Mittel, z.B. Aromastoffe, wie Vanillin oder Orangenextrakt, enthalten. Sie können außerdem Suspendierhilfsstoffe oder Dickungsmittel, wie Natriumcarboxymethylcellulose, Netzmittel, beispielsweise Kondensationsprodukte von Fettalkoholen mit Ethylenoxid, oder Schutzstoffe, wie p-Hydroxybenzoate, enthalten.

Injektionslösungen werden in üblicher Weise, z.B. unter Zusatz von Konservierungsmitteln, wie p-Hydroxybenzoaten, oder Stabilisatoren, wie Alkalisalzen der Ethylendiamintetraessigsäure und unter Zugabe geeigneter Lösungsvermittler hergestellt und in Injektionsflaschen oder Ampullen abgefüllt.

Die einen oder mehrere Wirkstoffe beziehungsweise Wirkstoffkombinationen enthaltenden Kapseln können beispielsweise hergestellt werden, indem man die Wirkstoffe mit inerten Trägern, wie Milchzucker oder Sorbit, mischt und in Gelatinekapseln einkapselt.

Die folgenden Beispiele dienen zur näheren Erläuterung der Erfindung:

Beispiel 1

N-[N-(1(S)-Ethoxycarbonyl-3-phenylpropyl)-L-lysyl]-4,5,6,7-tetrahydro-thieno[3,2-c]pyridin-4(S)-carbonsäure

a) N-(1-Ethoxycarbonyl-3-phenylpropyl)-L-$N^{\epsilon}$-t-butyl-oxycarbonyl-lysin

Zu einer Lösung aus 247,5 g 2-Oxo-4-phenylbuttersäureethylester und 74,2 g $N^{\epsilon}$-t-Butyloxycarbonyl-L-lysin in 1800 ml 50%igem Ethanol werden innerhalb von 3 1/2 Stunden 37,7 g $NaCNBH_3$ in 300 ml Ethanol zugetropft und die Lösung anschließend 12 Stunden bei Raumtemperatur gerührt. Ethanol wird im Vakuum abgedampft und die Wasserphase mit 1 n Natronlauge auf pH 9 gestellt und dreimal mit Ether extrahiert. Die Wasserphase wird dann mit 1 n HCl auf pH 4 gestellt und dreimal mit Essigester extrahiert. Die vereinigten Essigesterextrakte werden über $MgSO_4$ getrocknet und im Vakuum eingeengt. Man erhält 116 g hellgelbes Öl (88 % d.Th.).

b) N-[N-(1(S)-Ethoxycarbonyl-3-phenylpropyl)-L-$N^{\epsilon}$-t-butyloxycarbonyl-lysyl]-4,5,6,7-tetrahydro-thieno-[3,2-c]pyridin-4(S)-carbonsäure-t-butylester

Zu einer auf 0°C gekühlten Lösung von 4,3 g N-(1-Ethoxycarbonyl-3-phenylpropyl)-L-$N^{\epsilon}$-t-butyloxycarbonyl-lysin, 3,3 g 4,5,6,7-Tetrahydro-thieno-[3,2-c]pyridin-4-carbonsäure-t-butylester, 2,1 g 1-Hydroxybenzotriazol und 2 ml Triethylamin in 40 ml Tetrahydrofuran werden 3,5 g Dicyclohexylcarbodiimid gegeben und 1 Stunde bei 0°C und 12 Stunden bei Raumtemperatur gerührt. Nach Filtration wird die Lösung im Vakuum eingeengt, der Rückstand in Essigester aufgenommen, nacheinander mit $10^{-3}$n HCl, $KHCO_3$-Lösung und Wasser gewaschen, über $MgSO_4$ getrocknet und eingeengt. Das Öl wird über Kieselgel (Laufmittel: Essigester/n-Hexan (1:1) chromatographiert. Die Fraktionen mit dem $R_F$-Wert von 0,5 -0,65 werden zusammen über eine HPLC-Chromatographie - (Polygosil 60-2540 der Firma Machery-Nagel) mit Essigester/n-Hexan (1:1) gereinigt. Die Fraktionen mit dem $R_F$-Wert 0,56 enthalten 1,8 g N-[N-(1(S)-Ethoxycarbonyl-3-phenylpropyl)-L-$N^{\epsilon}$-t-butyloxycarbonyl-lysyl]-4,5,6,7-tetrahydro-thieno-[3,2-c]pyridin-4(S)-carbonsäure-t-butylester.

c) N-[N-(1(S)-Ethoxycarbonyl-3-phenylpropyl)-L-lysyl]-4,5,6,7-tetrahydro-thieno[3,2-c]pyridin-4(S)-carbonsäure

1,8 g der unter b) erhaltenen Verbindung werden in 200 ml 1 n HCl in Eisessig 30 Minuten bei Raumtemperatur gerührt. Der Eisessig wird im Vakuum abdestilliert, der Rückstand mit Isopropanol/Ether behandelt und der kristalline Niederschlag abfiltriert, gewaschen und getrocknet. Es werden 1,1 g (80 % d.Th.) des Dihydrochlorids der Titelverbindung erhalten.

Fp.: 162°C (Zers.)

Beispiel 2

N-[N-(1(S)-Carboxy-3-phenylpropyl)-L-lysyl]-4,5,6,7-tetrahydro-thieno[3,2-c]pyridin-4(S)-carbonsäure

a) N-[N-(1(S)-Ethoxycarbonyl-3-phenylpropyl)-L-$N^{\epsilon}$-t-butyloxycarbonyl-lysyl]-4,5,6,7-tetrahydro-thieno-[3,2-c]pyridin-4(S)-carbonsäuremethylester

Zu einer auf 0°C gekühlten Lösung von 8,7 g N-(1-Ethoxycarbonyl-3-phenylpropyl)-L-$N^{\epsilon}$-t-butyloxycarbonyl-lysin, 4,6 g 4,5,6,7-Tetrahydro-thieno-[3,2-c]pyridin-4-carbonsäuremethylester-hydrochlorid, 3,06 g 1-Hydroxybenzotriazol und 5,6 ml Triethylamin in 75 ml Dimethylformamid/Tetrahydrofuran (1:1) werden 4,5 g Dicyclohexylcarbodiimid gegeben und 1 Stunde bei 0°C und 12 Stunden bei Raumtemperatur gerührt. Nach Filtration wird die Lösung im Vakuum eingeengt, der Rückstand in Essigester aufgenommen, nacheinander mit $10^{-3}$ n HCl, $KHCO_3$-Lösung und Wasser gewaschen, über $MgSO_4$ getrocknet und eingeengt. Das Öl wird über Kieselgel (Laufmittel: Essigester/n-Hexan (1:1)) chromatographiert. Fraktionen mit einem $R_F$-Wert von 0,4 werden zusammen über eine HPCL-Chromatographie (Polygosil 60-2540 der Firma Machery-Nagel) mit Essigester/n-Hexan (1:1) gereinigt. Die Fraktionen mit dem $R_F$-Wert 0,38 enthalten 2,3 g N-[N-(1(S)-Ethoxycarbonyl-3-phenylpropyl)-L-$N^{\epsilon}$-t-butyloxycarbonyl-lysyl]-4,5,6,7-tetrahydro-thieno-[3,2-c]pyridin-4(S)-carbonsäuremethylester.

b) N-[N-(1(S)-Carboxy-3-phenylpropyl)-L-lysyl]-4,5,6,7-tetrahydro-thieno[3,2-c]pyridin-4(S)-carbonsäure

2,3 g der unter a) erhaltenen Verbindung werden mit 12 ml 1 n Natronlauge in 20 ml Acetonitril 12 Stunden bei Raumtemperatur gerührt. Das Acetonitril wird im Vakuum abdestilliert, der wäßrige Rückstand mit Essigester extrahiert, mit 1 n HCl neutralisiert und der Niederschlag abfiltriert, gewaschen und getrocknet. Die erhaltenen Kristalle werden mit 20 ml 1 n HCl in Eisessig 1/2 Stunde bei Raumtemperatur gerührt. Der Eisessig wird im Vakuum abdestilliert, der Rückstand mit Isopropanol/Ether ausgefällt, abgesaugt und getrocknet.

Es werden 1,46 g (71 % d.Th.) der Titelverbindung als farbloses amorphes Pulver erhalten.

Fp.: 175 -178°C (Zers.)

Beispiel 3

N-[N-(1(S)-Ethoxycarbonyl-3-phenylpropyl)-L-lysyl]-4,5,6,7-tetrahydro-thieno[2,3-c]pyridin-7(S)-carbonsäure

Wie unter Beispiel 1 beschrieben wurden aus 8,6 g N-(1-Ethoxycarbonyl-3-phenylpropyl)-L-$N^{\epsilon}$-t-butyloxy-carbonyl-lysin und 6,6 g 4,5,6,7-Tetrahydro-thieno-[2,3-c]pyridin-7-carbonsäure-t-butylester 2,3 g der Titelverbindung als Dihydrochlorid erhalten.

Beispiel 4

N-[N-(1(S)-Carboxy-3-phenylpropyl)-L-lysyl]-4,5,6,7-tetrahydro-thieno[2,3-c]pyridin-7(S)-carbonsäure

Aus 4,4 g N-(1-Ethoxycarbonyl)-3-phenylpropyl)-L-$N^{\epsilon}$-t-butyloxycarbonyl-lysin und 2,3 g 4,5,6,7-Tetrahydro-thieno[2,3-c]pyridin-7-carbonsäuremethylester-hydrochlorid werden wie unter Beispiel 2 beschrieben 0,9 g der Titelverbindung als Dihydrochlorid erhalten.

Fp.: 175°C (Zers.)

Beispiel 5

N-[N-(1(S)-Carboxy-3-phenylpropyl)-L-lysyl]-2,3,4,9-tetrahydro-1H-pyrido[3,4-b]indol-3(S)-carbonsäure

Aus 4,4 g N-(1-Ethoxycarbonyl-3-phenylpropyl)-L-$N^{\epsilon}$-t-butyloxycarbonyl-lysin und 2,7 g 2,3,4,9-Tetrahydro-1H-pyrido[3,4-b]indol-3(S)-carbonsäuremethylester-hydrochlorid werden, wie unter Beispiel 2 beschrieben, 0,8 g der Titelverbindung als Dihydrochlorid erhalten.

Fp.: 188°C (Zers.)

Beispiel 6

N-[N-(1(S)-Carboxy-3-phenylpropyl)-L-lysyl]-4,5,6,7-tetrahydro-1H-imidazo[4,5-c]pyridin-6(S)-carbonsäure

Aus 4,4 g N-(1-Ethoxycarbonyl-3-phenylpropyl)-L-$N^{\epsilon}$-t-butyloxycarbonyl-lysin und 2,2 g 4,5,6,7-Tetrahydro-1H-imidazo[4,5-c]pyridin-6(S)-carbonsäuremethylester-hydrochlorid werden, wie unter Beispiel 2 beschrieben, 2,3 g der Titelverbindung als Trihydrochlorid erhalten.

Fp.: 105°C (Zers.)

Beispiel 7

N-[N-(1(S)-Carboxy-3-phenylpropyl)-L-lysyl]-4,5,6,7-tetrahydro-thieno[2,3-c]pyridin-5(S)-carbonsäure

Aus 11,2 g N-(1-Ethoxycarbonyl-3-phenylpropyl)-L-$N^{\epsilon}$-t-butyloxycarbonyl-lysin und 6,0 g 4,5,6,7-Tetrahydrothieno[2,3-c]pyridin-5-carbonsäuremethylester-hydrochlorid werden, wie unter Beispiel 2 beschrieben, 1,5 g der Titelverbindung als Dihydrochlorid erhalten.

Fp.: 170°C (Zers.)

Beispiel 8

N-[N-(1(S)-Carboxy-3-phenylpropyl)-L-lysyl]4,5,6,7-tetrahydro-thieno[3,2,-c]pyridin-6(S)-carbonsäure

Aus 5,5 g N-(1-Ethoxycarbonyl-3-phenylpropyl)-L-$N^{\epsilon}$-t-butyloxycarbonyl-lysin und 3 g 4,5,6,7-Tetrahydrothieno[3,2-c]pyridin-6-carbonsäuremethylester-hydrochlorid werden, wie unter Beispiel 2 beschrieben, 0,8 g der Titelverbindung als Dihydrochlorid erhalten.

Fp.: 170°C (Zers.)

Beispiel 9

N-[N-(1(S)-Ethoxycarbonyl-3-phenylpropyl)-L-lysyl]-4,5,6,7-tetrahydro-thieno[2,3-c]pyridin-5(S)-carbonsäure

Aus 8,6 g N-(1-Ethoxycarbonyl-3-phenylpropyl)-L-$N^{\epsilon}$-t-butyloxycarbonyl-lysin und 6,6 g 4,5,6,7-Tetrahydrothieno[2,3-c]pyridin-5-carbonsäure-t-butylester werden, wie unter Beispiel 1 beschrieben, 2,0 g der Titelverbindung als Dihydrochlorid erhalten.

Fp.: 106 -109°C

Beispiel 10

N-[N-(1(S)-Carboxy-3-phenylpropyl)-L-lysyl]-4,5,6,7-tetrahydro-thieno[3,2-c]pyridin-6(S)-carbonsäure

Das wie in Beispiel 1 unter a) erhaltene N-(1-(R,S)-Ethoxycarbonyl-3-phenylpropyl)-L-$N^{\epsilon}$-tert.-butyloxycarbonyl-lysin wird in Essigester aufgenommen und mit wasserfreiem Diethylether versetzt. Die über Nacht ausgefallenen Kristalle werden abfiltriert und getrocknet. Man erhält ca. 60% der Theorie,

N-(1(S)-Ethoxycarbonyl-3-phenylpropyl)-2(S)-$N^{\epsilon}$-tert.-butyloxycarbonyl-lysin als farblose Kristalle.

4,4 g dieser Verbindung werden mit 2,2 g 4,5,6,7-Tetrahydro-thieno[3,2-c]pyridin-6(S)-carbonsäure-methylester-hydrochlorid wie in Beispiel 2 beschrieben, umgesetzt. Abweichend von dieser Vorschrift wird über Kieselgel mit Essigester/n-Hexan (1:1) gereinigt. Es werden 5,2 g (84 % der Theorie) N-[N-1(S)-Ethoxy-carbonyl-3-phenylpropyl)-L-lysyl]-4,5,6,7-tetrahydro-thieno-[3,2-c]pyridin-6(S)-carbonsäuremethylester als farbloses Öl erhalten. Diese Verbindung wird wie unter Beispiel 2 b) beschrieben zu 3,0 g (75 % der Theorie) der Titelverbindung umgesetzt.

Fp. 170 °C (Zers.).

Beispiel 11

N-[N-(1(S)-Carboxy-3-phenylpropyl)-L -lysyl-4,5,6,7-tetrahydro-thieno[3,2-c]pyridin-6(R)-carbonsäure

Wie unter Beispiel 10 beschrieben werden aus 2,2 g N-(N-(1-(S)-Ethoxycarbonyl-3-phenylpropyl)-L -$N^{\epsilon}$-tert.-butyloxycarbonyl-lysin und 1,1 g 4,5,6,7-Tetrahydro-thieno[3,2-c]pyridin-6(R)-carbonsäuremethylester-hydrochlorid 1,4 g der Titelverbindung als farblose Kristalle dargestellt.

Beispiel 12

N-[N-(1(S)-Carboxy-3-phenylpropyl)-L -$N^{\epsilon}$-tert.-butyloxycarboyl-lysyl]-4,5,6,7-tetrahydro-thieno[3,2-c]-pyridin-6(S)-carbonsäure.

7,8 g wie in Beispiel 10 beschrieben, erhaltenes N-[N-(1-(S)-Ethoxycarbonyl-3-phenylpropyl)-L -$N^{\epsilon}$-tert.-butyl-oxycarbonyl-lysyl]-4,5,6,7-tetrahydrothieno[3,2-c]pyridin-6(S)-carbonsäuremethylester werden in 60 ml Acetonitril (1 n Natronlauge (1:1) 12 Stunden bei Raumtemperatur gerührt. Acetonitril wird abdestilliert, der wäßrige Rückstand mit Essigester extrahiert, mit 1 n HCl neutralisiert, die ausgefallenen Kristalle abfiltriert, mit Diethylether gewaschen und getrocknet. 6,2 g farblose Kristalle (90 % der Theorie).

Fp. 123 -125 °C.

Beispiel 13 (Verfahren b)

a) $N^{\alpha}$-Benzyloxycarbonyl-$N^{\epsilon}$-t-butyloxycarbonyl-L-lysyl-L-spinacinmethyl-ester

760 mg $N^{\alpha}$-Benzyloxycarbonyl-$N^{\epsilon}$-t-butyloxycarbonyl-L-lysin, 436 mg L-Spinacinmethylesterhydrochlorid,456 mg Dicyclohexylcarbodiimid, 270 mg 1-Hydroxybenzotriazol und 200 mg Triethylamin werden in 15 ml wasserfreiem Dimethylformamid 2 Stunden bei Raumtemperatur gerührt. Es wird mit $KHSO_4$-, $NaHCO_3$-Lösung und Wasser jeweils zweimal gewaschen, über $MgSO_4$ getrocknet und im Vakuum eingedampft. Farbloses Öl, 870 mg (80 % der Theorie).

b) $N^{\epsilon}$-t-Butyloxycarbonyl-L-lysyl-L-spinacin-methylester

870 mg der unter a) erhaltenen Vebindung werden mit 100 mg Palladium-Kohle in 20 ml Methanol hydriert. Nach vollständiger Wasserstoffaufnahme wird der Katalysator abfiltriert und das Filtrat zur Trockne eingedampft.

c) N-[N-(1(R,S)-Ethoxycarbonyl-3-phenylpropyl)-N $^{\epsilon}$-t-butyloxycarbonyl-L-lysyl]-spinacinmethylester

Zu 650 mg der unter b) erhaltenen Verbindung und 1,65 g 2-Oxo-4-Phenyl-buttersäureethylester in 10 ml Ethanol wird eine Lösung von 300 mg Natriumcyanborhydrid in 5 ml Ethanol zugetropft. Es wird im Vakuum eingedampft, der Rückstand in Essigester aufgenommen und mit $10^{-3}$n HCl und Wasser gewaschen. Nach Trocknen über $MgSO_4$ wird im Vakuum eingedampft und der ölige Rückstand über Kieselgel (Laufmittel Essigester/n-Hexan (1:1) chromatographiert. RF-Wert = 0,6. Es wurden 770 mg farbloses Öl erhalten.

d) N-[N-1(R,S)-Carboxy-3-phenylpropyl)-L-lysyl]-L-spinacin

Aus 770 mg der unter c) erhaltenen Verbindung werden wie unter Beispiel 2 beschrieben 620 mg als Trihydrochlorid der Titelverbindung erhalten.

Fp. 95°C.

Beispiel 14 (Verfahren b)

N-[N-(1(S)-Carboxy-3-phenylpropyl-L-lysyl]-4,5,6,7-tetrahydro-1H-imidazo[4,5-c]pyridin-6(S)-carbonsäure

2,05 g $N^{\epsilon}$-t-Butyloxycarbonyl-L-lysyl-L-spinacinmethylester werden in 50 ml Trifluorethanol mit 1,36 g 2-Brom-4-phenylbuttersäureethylester und 530 mg Natriumcarbonat über Nacht bei Raumtemperatur gerührt. Trifluorethanol wird im Vakuum abgezogen, der Rückstand mit Essigester aufgenommen, mit $10^{-3}$n HCl und Wasser gewaschen, über $MgSO_4$ getrocknet und im Vakuum eingedampft. Der ölige Rückstand wird über Kieselgel mit Essigester/n-Hexan (1:1) chromatographiert.

Rf = 0,6. Es werden 2,2 g als farbloses Öl erhalten.

Durch Hydrolyse entsprechend Beispiel 2 (b) erhält man die Titelverbindung, Fp. 105°C (Zers.).

Pharmazeutische Anwendungsbeispiele

a) Dragees

| 1 Drageekern enthält: | |
|---|---|
| Wirkstoff gemäß Anspruch 1 | 20,0 mg |
| Milchzucker | 100,0 mg |
| Maisstärke | 75,0 mg |
| Gelatine | 3,0 mg |
| Magnesiumstearat | 2,0 mg |
| | 200,0 mg |

Herstellung:

Die Mischung der Wirksubstanz mit Milchzucker und Maisstärke wird mit einer 10%igen wäßrigen Gelatinelösung durch ein Sieb mit 1 mm Maschenweite granuliert, bei 40°C getrocknet und nochmals durch ein Sieb gerieben. Das so erhaltene Granulat wird mit Magnesiumstearat gemischt und verpreßt. Die so erhaltenen Kerne werden in üblicher Weise mit einer Hülle überzogen, die mit Hilfe einer wäßrigen Suspension von Zucker, Titandioxyd, Talkum und Gummi arabicum aufgebracht wird. Die fertigen Dragees werden mit Bienenwachs poliert.

b) Tabletten

| | |
|---|---|
| Wirkstoff gemäß Anspruch 1 | 50,0 mg |
| Milchzucker | 100,0 mg |
| Maisstärke | 70,0 mg |
| lösliche Stärke | 7,0 mg |
| Magnesiumstearat | 3,0 mg |
| | 230,0 mg |

Herstellung:

Wirkstoff und Magnesiumstearat werden mit einer wäßrigen Lösung der löslichen Stärke granuliert, das Granulat getrocknet und innig mit Milchzucker und Maisstärke vermischt. Das Gemisch wird sodann zu Tabletten von 230 mg Gewicht verpreßt, die je 100 mg Wirkstoff enthalten.

c) Injektionslösungen

| | | | |
|---|---|---|---|
| Wirkstoff gemäß Anspruch 1 | | 10,0 | mg |
| Ethanolamin | | 60,0 | mg |
| Natriumchlorid | | 20,0 | mg |
| destilliertes Wasser | ad | 2 | ml |

Herstellung:

Der Wirkstoff und die Hilfsstoffe werden in einer ausreichenden Menge destilliertem Wasser gelöst und mit der erforderlichen Menge Wasser auf die gewünschte Konzentration gebracht. Die Lösung wird filtriert und unter aseptischen Bedingungen in 2 ml Ampullen abgefüllt. Die Ampullen werden sterilisiert und verschlossen. Jede Ampulle enthält 50 mg Wirkstoff.

d) Kapseln

| | | |
|---|---|---|
| Wirkstoff gemäß Anspruch 1 | 20,0 | mg |
| Milchzucker | 230,0 | mg |
| Maisstärke | 40,0 | mg |
| Talk | 10,0 | mg |
| | 300,0 | mg |

Herstellung:

Wirkstoff, Milchzucker und Maisstärke werden zunächst in einem Mischer und dann in einer Zerkleinerungsmaschine vermengt. Das Gemisch wird nochmals in den Mischer gegeben, gründlich mit dem Talk vermengt und maschinell in Hartgelatinekapseln abgefüllt.

e) Suppositorien

| | |
|---|---|
| Wirkstoff gemäß Anspruch 1 | 0,1 g |
| Kakaobutter (Fp. 36-37°C) | 1,6 g |
| Carnaubawachs | 0,1 g |
| | 1,8 g |

Herstellung:

Kakaobutter und Carnaubawachs werden geschmolzen, gründlich vermengt und auf 45°C abgekühlt. In diese Masse wird der feinpulverisierte Wirkstoff eingerührt. Anschließend wird die Mischung in leicht vorgekühlten Suppositorienformen geeigneter Größe gegossen und abkühlen gelassen.

## Ansprüche

1. Neue Aminosäure-Derivate der allgemeinen Formel

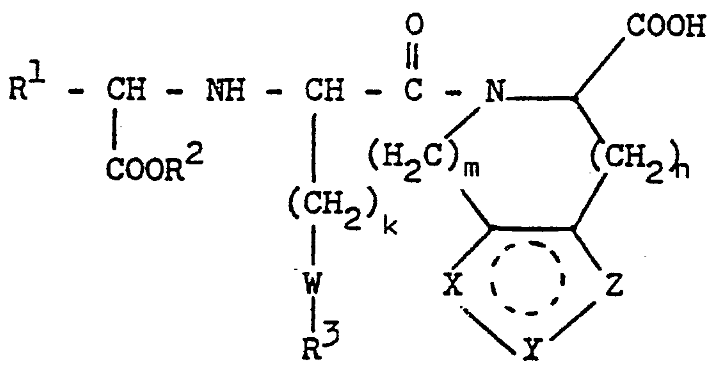

(I)

in der

$R^1$ Wasserstoff oder eine ggf. phenylsubstituierte Alkylgruppe mit 1 bis 6 Kohlenstoffatomen;

$R^2$ Wasserstoff oder eine ggf. phenylsubstituierte Alkylgruppe mit 1 bis 4 Kohlenstoffatomen;

$R^3$ Wasserstoff, eine Alkylgruppe mit 1 bis 7 Kohlenstoffatomen, die endständig eine Aminogruppe oder einen Phenylring tragen kann, oder die Gruppe $R^4$ -CO -;

$R^4$ eine Alkyl-oder Alkoxygruppe mit 1 bis 5 Kohlenstoffatomen, die im Falle W = NH, in $\alpha$-Stellung eine Aminogruppe tragen kann;

W Sauerstoff, Schwefel oder die NH-Gruppe;

n und m jeweils 0,1 oder 2, wobei die Summe n und m 1 oder 2 ist;

k 1, 2, 3 oder 4;

X, Y und Z Sauerstoff, Schwefel, $NR^5$, $CR^6$, $CHR^6$,

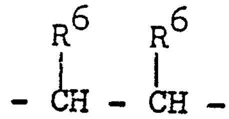 oder 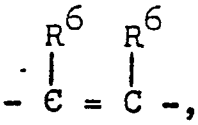,

mit der Maßgabe, daß nur einer der Reste X, Y und Z O, S,

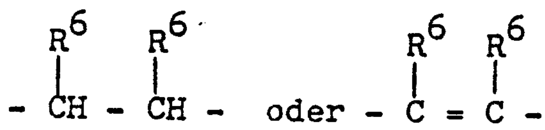

und ein oder zwei der Reste X, Y und Z $NR^5$ bedeuten können;

$R^5$ Wasserstoff oder eine Alkylgruppe mit 1 bis 4 Kohlenstoffatomen und

$R^6$ Wasserstoff oder zusammen mit einem vicinal stehenden Rest $R^6$ einen Phenylring bedeutet, und ihre Salze.

2. Verbindungen nach Anspruch I entsprechend der Formel

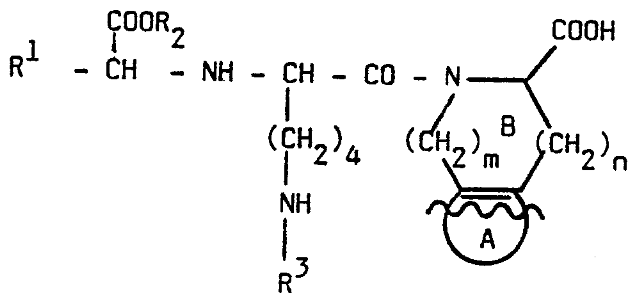

(Ia)

in der

$R^2$, $R^3$ die obige Bedeutung haben,

m 1 oder 2,

n 0 oder 1 bedeutet, wobei die Summe m + n gleich 2 ist,

A für einen der Reste

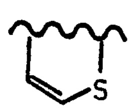

,

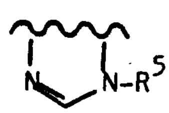

oder

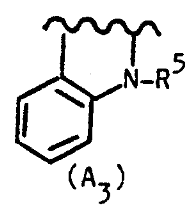

$(A_1)$ $(A_2)$ $(A_3)$

steht, wobei die Reste $A_1$, $A_2$ und $A_3$ auf beiderlei Weise mit dem Ring B verknüpft sein können, und

$R^5$ wie oben definiert ist.

3. Verbindungen nach Anspruch 1 entsprechend der Formel

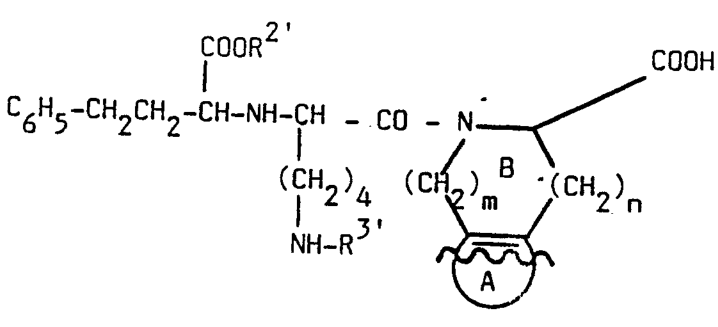

(Ib)

in der

$R^{2'}$ H oder $C_1$-$C_4$-Alkyl und

$R^{3'}$ H oder $COR^4$

bedeutet, wobei $R^4$ wie oben definiert ist,

m 1 oder 2,

n 0 oder 1 bedeutet, wobei die Summe m + n gleich 2 ist, und

A' für einen der Reste

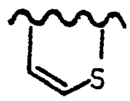

,

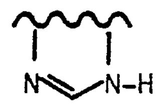

oder

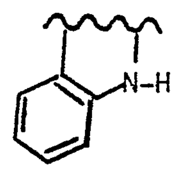

$(A_1)$ $(A_{2'})$ $(A_{3'})$

steht, wobei die Reste $A_1$, $A_{2'}$ und $A_{3'}$ auf beiderlei Weise mit dem Ring B verknüpft sein können.

4. Verbindungen nach Anspruch 1, 2 oder 3, dadurch gekennzeichnet, daß alle Asymmetriezentren in der L-Form vorliegen.

5. N-[N-(1(S)-Ethoxycarbonyl-3-phenylpropyl)-L-lysyl]-4,5,6,7-tetrahydro-thieno[3,2-c]pyridin-4(S)-carbonsäure.

6. N-[N-(1(S)-Ethoxycarbonyl-3-phenylpropyl)-L-lysyl]-4,5,6,7-tetrahydro-thieno[2,3-c]pyridin-7(S)-carbonsäure.

7. N-[N-(1(S)-Carboxy-3-phenylpropyl)-L-lysyl]-4,5,6,7-tetrahydro-thieno[2,3-c]pyridin-5(S)-carbonsäure.

8. N-[N-(1(S)-Carboxy-3-phenylpropyl)-L-lysyl]-4,5,6,7-tetrahydro-thieno[3,2-c]pyridin-6(S)-carbonsäure.

9. N-[N-(1(S)-Carbethoxy-3-phenylpropyl)-L-lysyl]-4,5,6,7-tetrahydro-thieno[3,2-c]pyridin-5(S)-carbonsäure.

10. N-[N-(1(S)-Carbethoxy-3-phenylpropyl)-L-lysyl]-4,5,6,7-tetrahydro-thieno[3,2-c]pyridin-6(S)-carbonsäure.

10a. N-[N-(1(S)-Carboxy-3-phenylpropyl)-L-lysyl]-4,5,6,7-tetrahydro-thieno[3,2-c]pyridin-6(R)-carbonsäure

11. Verfahren zur Herstellung neuer Aminosäure-Derivate der allgemeinen Formel I gemäß Anspruch 1 und ihre Salze, dadurch gekennzeichnet, daß man

a) eine Verbindung der allgemeinen Formel II

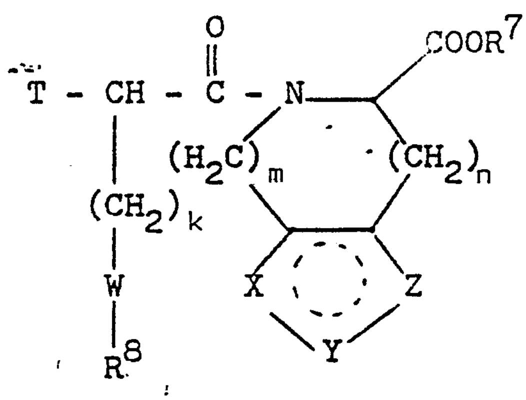

(II)

mit einer Verbindung der allgemeinen Formel III

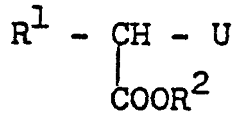

(III)

in denen

T eine nucleofuge Gruppe und

U eine Aminogruppe oder umgekehrt T eine Aminogruppe und U eine nucleofuge Gruppe,

$R^1$ Wasserstoff, eine Alkylgruppe mit 1 bis 4 Kohlenstoffatomen, die Benzylgruppe oder die Trimethylsilylgruppe,

$R^8$ die Bedeutung von $R^3$ oder, im Falle, daß W für -NH oder W für -S -und $R^3$ für -H steht, eine der Schutzgruppen tert. Butyloxycarbonyl, Benzyloxycarbonyl, Fluorenylmethyloxycarbonyl, Benzyl oder Trityl bedeutet und die Reste $R^1$, $R^2$, $R^3$, W, k, n, m, X, Y und Z die oben angeführte Bedeutung besitzen, umsetzt, oder daß man

b) ein α-Oxocarbonsäurederivat der allgemeinen Formel

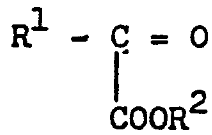

(IV)

worin $R^1$ und $R^2$ die oben genannte Bedeutung haben, mit einem unter a) beschriebenen Aminosäureamid der allgemeinen Formel II ($T = NH_2$) zum entsprechenden Imin umsetzt und dieses reduziert, oder daß man

c) eine Verbindung der allgemeinen Formel

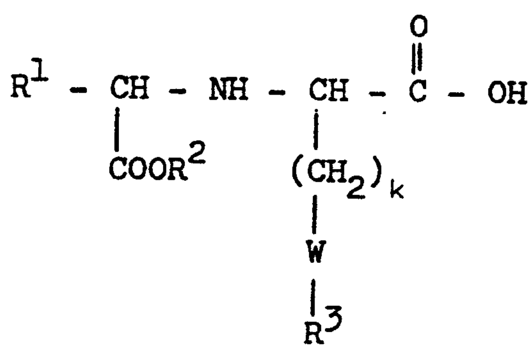

(V)

worin $R^1$, $R^2$, $R^3$, W und k die oben angegebene Bedeutung besitzen, mit einer Aminosäure der allgemeinen Formel

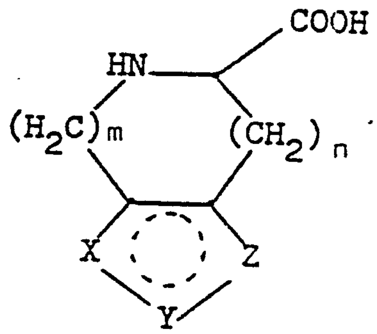

(VI)

worin die Reste X, Y und Z sowie n und m die oben angegebene Bedeutung haben, beziehungsweise mit einem Ester einer Verbindung der allgemeinen Formel VI ($R^7$ anstelle von Wasserstoff) kondensiert, wobei für W = NH und W = S und $R^3$ = H das Wasserstoffatom durch eine der Schutzgruppen $R^8$ ersetzt werden muß, und daß man, sofern in den nach a) bis c) erhaltenen Verbindungen $R^7$ und/oder $R^8$ eine andere Bedeutung als Wasserstoff haben, die betreffenden Gruppen $R^7$ und/oder $R^8$ hydrolytisch entfernt und daß man gewünschtenfalls ein so erhaltenes Endprodukt der allgemeinen Formel I nach üblichen Verfahren in ein Salz umwandelt.

12. Pharmazeutische Zubereitungen, enthaltend als Wirkstoff eine oder Verbindungen der Formel I oder ihre Salze in Kombination mit üblichen Hilfs-und/oder Trägerstoffen.

13. Pharmazeutische Zubereitungen, enthaltend als Wirkstoff eine oder mehrere Verbindungen der allgemeinen Formel I oder ihre Salze in Gemisch mit bekannten Saluretika beziehungsweise Diuretika und/oder Antihypertonika.

14. Verwendung der Verbindungen nach Anspruch 1 bei der Behandlung des Bluthochdruckes, bei der Cardioprotektion bzw. Infarktprophylaxe oder der Ökonomisierung des myocardialen Sauerstoffverbrauchs.